# EUROPEAN PATENT APPLICATION

(11) **EP 3 698 757 A1**
(43) Date of publication of application: **26.08.2020**
(21) Application number: 20275047.7
(22) Date of filing: 20.02.2020
(51) Int. Cl.: A61F 2/07, A61F 2/848, A61F 2/91

(54) **HYBRID STENT DESIGNS**

(30) Priority: 21.02.2019 US 201962808608 P
(71) Applicant: Cook Medical Technologies LLC, Bloomington, IN 47404 (US)
(72) Inventor: YANG, Shuo, West Lafayette, IN 47906 (US)
(74) Representative: Williams Powell

(57) **Abstract**

The present embodiments provide a stent comprising a proximal segment (50) having a zig-zag portion (52), which comprises a plurality of proximal and distal apices separated by a plurality of angled strut segments. A distal segment (60) may comprise a connection strut, where the connection strut comprises a bent segment extending between adjacent distal apices of the zig-zag portion (50). The stent may comprise at least one diamond-shaped closed cell, which is bounded proximally by first and second angled strut segments of the zig-zag portion meeting at a first proximal apex of the zig-zag portion, and further bounded distally by the connection strut having the bent segment. At least one distal open region may be located distally beneath a second proximal apex of the zig-zag portion that is adjacent to the first proximal apex of the zig-zag portion. The at least one diamond-shaped closed cell may comprise an asymmetric shape.

## Description

The present embodiments relate generally to apparatus and methods for treating medical conditions, and more specifically, to stents and stent-grafts for use in body vessels to treat those medical conditions.

### BACKGROUND

Stents may be inserted into an anatomical vessel or duct for various purposes. Stents may maintain or restore patency in a formerly blocked or constricted passageway, for example, following a balloon angioplasty procedure. Other stents may be used for different procedures, for example, stents placed in or about a graft have been used to hold the graft in an open configuration to treat an aneurysm. Additionally, stents coupled to one or both ends of a graft may extend proximally or distally away from the graft to engage a healthy portion of a vessel wall away from a diseased portion of an aneurysm to provide endovascular graft fixation.

Stents may be either self-expanding or balloon-expandable, or they can have characteristics of both types of stents. Self-expanding stents may be delivered to a target site in a compressed configuration and subsequently expanded by removing a delivery sheath, removing trigger wires and/or releasing diameter reducing ties. With self-expanding stents, the stents expand primarily based on their own expansive force without the need for further mechanical expansion. In a stent made of a shape-memory alloy such as nitinol, the shape-memory alloy may be employed to cause the stent to return to a predetermined configuration upon removal of the sheath or other device maintaining the stent in its predeployment configuration.

When a stent-graft having at least one stent is deployed in a vessel, such as the aorta, and blood flows in a proximal to distal direction away from the heart, there is a possibility of "infolding" of graft material, particularly at the proximal end of the graft material. For example, if a stent-graft is deployed to treat an abdominal aortic aneurysm, blood flowing distally into the graft may pull the proximal edge of the graft in a radially inward direction, particularly if an optimal proximal seal is not achieved with the vessel wall. In this case, the graft material that becomes pulled inward may impede blood flow through the stent-graft lumen, or an endoleak may occur. Furthermore, if the proximal end of a stent-graft is deployed in a curved portion of a vessel, such as the aortic arch or thoracic aorta, it may be difficult to conform the proximal edge of the stent-graft to the curving vessel wall, which also may result in blood flow catching on the graft and potential endoleaks.

### SUMMARY

Aspects of the present invention seek to provide an improved stent.

According to an aspect of the invention, there is provided a stent as in claim 1.

According to an aspect of the invention, there is provided a stent as in claim 12.

The present embodiments provide stents and stent-grafts for use in medical procedures. In one embodiment, a stent may comprise a proximal segment comprising a zig-zag portion, which comprises a plurality of proximal and distal apices separated by a plurality of angled strut segments. The zig-zag portion may extend circumferentially around a full perimeter of the stent. A distal segment may comprise a connection strut, where the connection strut comprises a bent segment extending between adjacent distal apices of the zig-zag portion. The stent may comprise at least one diamond-shaped closed cell, which is bounded proximally by first and second angled strut segments of the zig-zag portion meeting at a first proximal apex of the zig-zag portion, and further bounded distally by the connection strut having the bent segment. At least one distal open region may be located distally beneath a second proximal apex of the zig-zag portion that is adjacent to the first proximal apex of the zig-zag portion.

In one example, in an expanded state, the at least one diamond-shaped closed cell may comprise an asymmetric shape, such that the first and second angled strut segments are different than a mirror image of the connection strut. In one embodiment, in an expanded state, a first axial length spanned between the proximal and distal apices of the zig-zag portion may be greater than a second axial length spanned by the connection strut when measured along a longitudinal axis of the stent.

In one example, at least one of the plurality of angled strut segments of the zig-zag portion may comprise a first thickness, which may be greater than a second thickness of the connection strut. Third and fourth angled strut segments of the zig-zag portion, which extend directly from the second proximal apex, may comprise thicknesses greater than the connection strut.

In one embodiment, the first and second angled strut segments associated with the diamond-shaped cells may be orientated at a first angle relative to a longitudinal axis of the stent in an expanded state, and third and fourth angled strut segments extending from the second proximal apex may be orientated at a second angle relative to the longitudinal axis of the stent in the expanded state. The first angle may be less than the second angle. The first angle may be between about 5 degrees and about 45 degrees, and the second angle may be between about 20 degrees and about 90 degrees.

In some embodiments, the connection strut may comprise one of a V-shape or a U-shape. In various embodiments, the stent may comprise alternating proximal apices having different features, where a first proximal apex comprises a bore sized to receive an imaging element, and a second proximal apex comprises at least one barb. In some embodiments, the stent may comprise at least one distal apex adapted to be coupled to a graft, the at least one distal apex comprising a suture bore, an imaging bore, and a barb.

Other systems, methods, features and advantages of embodiments of the invention will be, or will become, apparent to one with skill in the art upon examination of the following figures and detailed description. It is intended that all such additional systems, methods, features and advantages be within the scope of the invention, and be encompassed by the following claims.

### BRIEF DESCRIPTION OF THE DRAWINGS

Embodiments of the invention are described below, by way of example only, with reference to the accompanying drawings. The components in the figures are not necessarily to scale, emphasis instead being placed upon illustrating the principles of embodiments of the invention. Moreover, in the figures, like referenced numerals designate corresponding parts throughout the different views.
FIG. 1 is a perspective view of a stent according to a first embodiment in an expanded state.
FIG. 2 is a perspective view depicting the stent of FIG. 1 coupled to a proximal region of a graft.
FIG. 3 is a side perspective view illustrating features of a portion of the stent of FIG. 1.
FIGS. 4A-4B are side views depicting a symmetric stent and an asymmetric stent, respectively, in expanded states.
FIGS. 5A-5B are side views depicting exemplary compression of portions of the symmetric and asymmetric stents of FIGS. 4A-4B, respectively.
FIG. 6 is a perspective view of a stent according to a second embodiment in an expanded state.
FIG. 7 is a perspective view of a stent according to a third embodiment in an expanded state.

### DETAILED DESCRIPTION OF THE PREFERRED EMBODIMENTS

In the present application, the term "proximal end" is used when referring to that end of a medical device closest to the heart after placement in the human body of the patient, and may also be referred to as the inflow end (the end that receives fluid first), and the term "distal end" is used when referring to that end opposite the proximal end, or the one farther from the heart after its placement, and may also be referred to as the outflow end (that end from which fluid exits).

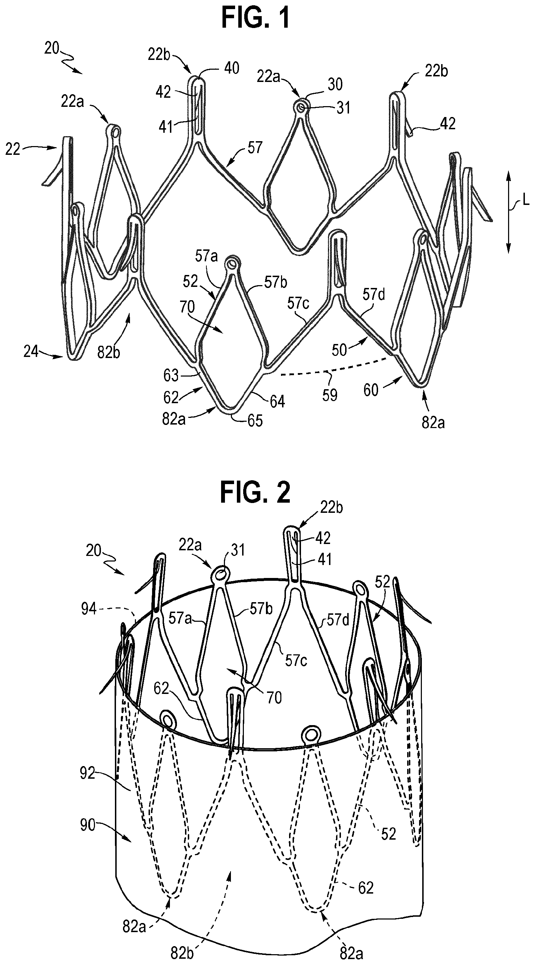

Referring to FIGS. 1-3, a first embodiment of a stent 20 is shown and described. The stent 20 may be manufactured into a cylindrical shape having proximal and distal ends 22 and 24, respectively. In one non-limiting example, the stent 20 may be formed from a continuous cylinder into which a pattern may be cut by a laser or by chemical etching to produce slits in the wall of the cylinder. The resulting structure may then be heat set to give it a desired final configuration. The preferred final configuration includes a shape having a series of proximal apices and a series of distal apices, as generally shown in FIGS. 1-3.

The stent 20 has a reduced diameter delivery state so that it may be advanced to a target location within a vessel or duct. The stent 20 also has an expanded deployed state to apply a radially outward force upon at least a portion of a vessel or duct, *e.g.,* to maintain patency within a passageway, or to hold open the lumen of a graft. In the expanded state, fluid flow is allowed through a central lumen of the stent 20.

The stent 20 may be manufactured from a super-elastic material. Solely by way of example, the super-elastic material may comprise a shape-memory alloy, such as a nickel titanium alloy (nitinol). If the stent 20 comprises a self-expanding material such as nitinol, the stent may be heat-set into the desired expanded state, whereby the stent 20 can assume a relaxed configuration in which it assumes the preconfigured first expanded inner diameter upon application of a certain cold or hot medium. In other embodiments, the stent 20 may be made from other metals and alloys that allow the stent 20 to return to its original, expanded configuration upon deployment, without inducing a permanent strain on the material due to compression. Solely by way of example, the stent 20 may comprise other materials such as stainless steel, cobalt-chrome alloys, amorphous metals, tantalum, platinum, gold and titanium. The stent 20 also may be made from non-metallic materials, such as thermoplastics and other polymers.

The stent 20 may be used alone or may be coupled to a graft, such as the graft 90 of FIG. 2. In one non-limiting example, described further below, the graft 90 may comprise a proximal region 92 guiding flow towards a distal region, and the stent 20 may be coupled to the proximal region 92. In some embodiments, the stent 20 may overlap with a proximal edge 94 of the graft 90, as depicted in FIG. 2. If the stent 20 is coupled to the graft 90 to form a stent-graft, the stent-graft may comprise multiple additional stents along its length. The stent-graft may have any number of stents having any variety of shapes, although it is preferred that the most proximal stent overlapping the graft 90 comprises at least one of the stents 20, 120 or 220 described herein. If the stent 20 is used as a stand-alone device, it may provide support to a vessel or duct without an attached graft material. In the latter embodiment, the stent 20 may be used to treat a wide range of conditions, including but not limited to arterial and biliary stenosis.

The proximal end 22 of the stent 20 may comprise multiple adjacent proximal apices 22a and 22b, while the distal end 24 of the stent 20 may comprise distal apices 82a separated by distal open regions 82b, as shown in FIGS. 1-3 and described further below.

One or more pairs of adjacent, proximal apices 22a and 22b may comprise different features. For example, a first proximal apex 22a may comprise an end region 30 having a bore 31 formed therein, where the bore 31 is configured to receive an imaging element. A second, adjacent proximal apex 22b comprises an end region 40 having an integral barb 42 formed therein, as best seen in FIGS. 1-3.

The barbs 42 may be formed by laser cutting a desired barb shape into the end regions 40. A slit 41 therefore is formed into each end region 40 after the desired barb shape is formed, as best seen in FIGS. 2-3. Once the desired barb shape is cut, a main body of the barb 42 may be bent in a radially outward direction with respect to the end region 40. The angle may comprise any acute angle, or may be substantially orthogonal or obtuse. If desired, the barbs 42 may be sharpened, for example, by grinding the tip of the barb, to facilitate engagement at a target tissue site.

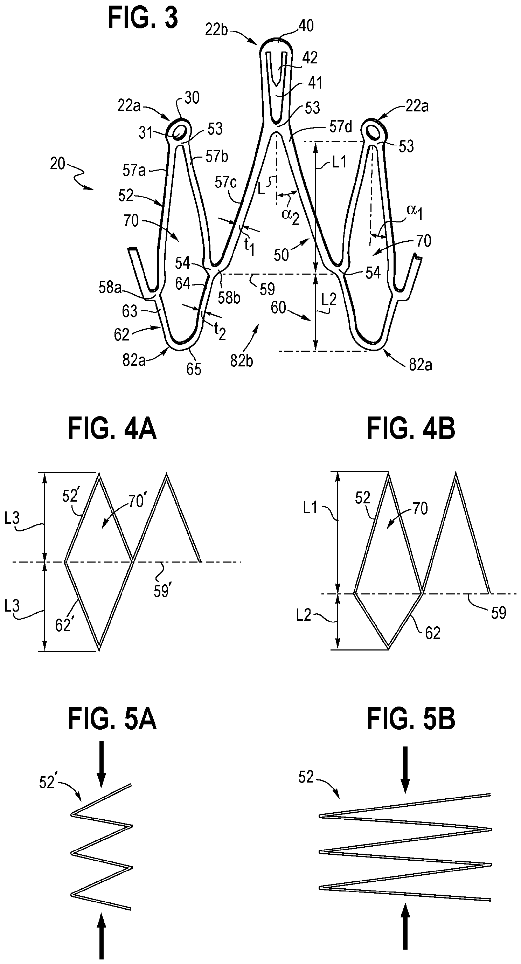

The stent 20 further generally comprises a proximal segment 50 and a distal segment 60, which are separated by a dividing line 59, as best seen in FIG. 1 and FIG. 3. A zig-zag portion 52 is disposed in the proximal segment 50. The zig-zag portion 52 has proximal apices 53, which transition into the end regions 30 and 40, and further has distal apices 54, as best seen in FIG. 3. A plurality of angled strut segments 57 continue around a perimeter of the zig-zag portion, thereby defining a perimeter of the proximal segment 50 of the stent 20. For example, a first angled strut segment 57a of the zig-zag portion 52 is disposed between a proximal apex 53 and a corresponding distal apex 54, and a second angled strut segment 57b is disposed between the same proximal apex 53 and an adjacent distal apex 54, with several more angled strut segments 57 spanning between proximal and distal apices 53 and 54. In this manner, the stent 20 may be formed into a continuous, generally cylindrical shape, as shown in FIGS. 1-3.

Expansion of the stent 20 is at least partly provided by the angled strut segments 57, which may be substantially parallel to one another in a compressed state (not shown), but tend to bow outward away from one another in the expanded state shown in FIGS. 1-3. In the compressed state, the first and second angled strut segments 57 may be compressed such that they are substantially parallel to one another along a longitudinal axis L of the stent 20.

The angled strut segments 57 meet with one another distally to form distal transition regions 58a and 58b, which are generally located at the dividing line 59, as best seen in FIG. 3. Each distal transition region 58a and 58b may comprise a larger surface area relative to the individual segments, since each distal transition region is composed substantially of multiple different angled segments 57 meeting up together.

The distal segment 60, which is disposed distal to the dividing line 59, comprises at least one connection strut 62. In one embodiment, the connection strut 62 comprises a generally bent shape, and may comprise a V-shape, a U-shape, or the like, as depicted in FIGS. 1-3. In this example, the connection strut 62 has first and second generally straight segments 63 and 64, respectively, and a bent segment 65 disposed therebetween, as seen in FIGS. 1-3.

The first generally straight segment 63 of the connection strut 62 connects with one of the distal apices 54 of the zig-zag portion 52 at a first distal transition region 58a, as best seen in FIG. 3. The second generally straight segment 64 of the connection strut 62 connects with another one of the distal apices 54 of the zig-zag portion 52 at a second distal transition region 58b, as further seen in FIG. 3.

In this manner, a plurality of diamond-shaped cells 70 are formed along the stent 20. Specifically, the diamond-shaped cells 70 are bounded on their four sides by first and second angled strut segments 57a and 57b of the zig-zag portion 52, together with the first and second generally straight segments 63 and 64 of the connection strut 62.

The upper vertex of each diamond-shaped cell 70 is located at a proximal apex 53 of the zig-zag portion 52 at alternating apices 22a of the broader stent 20. The lower vertex of each diamond-shaped cell 70 is located at the bent segment 65 of the connection strut 62, which is at distal apices 82a of the broader stent 20.

Notably, distal open regions 82b of the stent 20 are formed along the circumferential regions of the stent 20 coinciding with the alternating proximal apices 22b, which do not form diamond-shaped cells 70. Although third and fourth angled strut segments 57c and 57d extend from the second proximal apices 22b, the omission of the connection struts 62 in the areas beneath the second proximal apices 22b yields the distal open regions 82b, as shown in FIGS. 1-3.

In accordance with one embodiment, the first and second angled strut segments 57a and 57b coinciding with the plurality of diamond-shaped cells 70 may comprise a first angle **α₁** relative to the longitudinal axis **L** of the stent 20, as shown in FIG. 3. Further, the angled strut segments 57c and 57d outside of the plurality of diamond-shaped cells 70 comprise a second angle **α₂** relative to the longitudinal axis **L.** The first angle **α₁** is less than the second angle **α₂**. In this manner, the zig-zag portion 52 is not symmetrical around its circumference in terms of strut angles, but rather varies between strut segments having greater or lesser angles depending on whether such strut segments make up part of the diamond-shaped cells 70.

In one example, the first angle **α₁** is between about 5 degrees and about 45 degrees when measured in the manner shown in FIG. 3, while the second angle **α₂** is between about 20 degrees to about 90 degrees. If the first angle **α₁** is on the higher end of the spectrum, e.g., closer to 45 degrees, then it is contemplated that the second angle **α₂** will also be on the higher end of the spectrum, e.g., closer to 90 degrees, such that the first angle **α₁** remains less than the second angle **α₂** for a particular design.

Advantageously, this variable angular orientation of the first and second angled strut segments 57a-57d yields a balanced behavior of the stent 20 overall, particularly when in the expanded state. Specifically, the distal open regions 82b of the zig-zag portion 52 (aligning with proximal apices 22b) would ordinarily be weaker in radial strength, while the closed design of the diamond-shaped cells 70 would ordinarily be stronger in radial strength, all other factors being considered equal. By providing a greater second angle **α₂** in the open areas of otherwise weaker radial strength, and a smaller first angle **α₁** in the closed areas of otherwise greater radial strength, a more balanced radial force behavior is achieved around the perimeter of the stent 20.

In accordance with another embodiment, the thickness of struts of the stent 20 may vary in specific regions. In particular, some or all of the angled strut segments 57a-57d of the zig-zag portion 52 may comprise a first thickness **t₁,** while one or all of the segments 63-65 along the length of the connection strut 62 may comprise a second thickness **t₂,** when measured as shown in FIG. 3. The first thickness **t₁** is greater than the second thickness **t₂.**

Advantageously, the differing thicknesses **t₁** and **t₂** facilitate a balanced behavior of the stent 20 overall, particularly when in the expanded state. Specifically, the distal open regions 82b (aligning with proximal apices 22b) would ordinarily be weaker in radial strength, while the closed design of the diamond-shaped cells 70 would ordinarily be stronger in radial strength, all other factors being considered equal. By providing a greater thickness **t₁** in selected areas of otherwise weaker radial strength, and a smaller thickness **t₂** in selected areas of otherwise greater radial strength, a more balanced radial force behavior is achieved around the perimeter of the stent 20.

Notably, in this example, the angled strut segments 57c and 57d outside of the plurality of diamond-shaped cells 70 comprise the first thickness **t₁,** while the angled strut segments 57a and 57b coinciding with the plurality of diamond-shaped cells 70 may comprise either the first thickness **t₁**, the second thickness **t₂,** or a third thickness that is different. Moreover, it is contemplated that the thickness may vary along the connection strut 62 itself, although it will be advantageous that at least a portion of the connection strut 62 comprises one or more areas of reduced thickness **t₂** relative to the first thickness **t₁.**

As a related advantage, the areas with smaller thickness **t₂** take up less space in a compressed state, enabling a reduced overall delivery profile of the stent 20.

In accordance with yet another embodiment, an axial length **L₁** spanned by the proximal segment 50 is greater than an axial length **L₂** spanned by the distal segment 60, when measured along the longitudinal axis **L** of the stent and with respect to the dividing line 59, as shown in FIG. 3. In other words, the axial length spanned by the angled segments 57 is greater than the axial length spanned by the connection strut 62, when both are measured with respect to the main longitudinal axis **L.** In this manner, the diamond-shaped cells 70 comprise an asymmetric profile, whereby the two proximal struts (i.e., segments 57a and 57b of the zig-zag portion 52) are longer than the two distal struts (i.e., segments 63 and 64 of the connection strut 62). Advantageously, this differing lengths **L₁** and **L₂** may provide an enhanced ability to compress the stent 20 in the regions of the diamond-shaped cells 70.

FIGS. 4A-4B and FIGS. 5A-5B help illustrate such advantages. FIG. 4A depicts a hypothetical stent having a diamond-shaped cell 70' in which the axial length **L₃** above and below a dividing line 59' are approximately equal, i.e., the axial length of the zig-zag portion 52' is reduced while the axial length of the connection strut 62' is increased. As depicted in FIG. 5A, the design of such smaller axial length zig-zag portion 52' is harder to compress and the shorter strut length tends to be less stable, i.e., it may not retain its cylindrical shape during compression and has an increased likelihood of buckling out of plane.

In contrast, it can be seen that the asymmetric design of the present embodiments shown in FIG. 4B may be easier and more stable to compress as shown in FIG. 5B due to the longer axial length of the zig-zag portion 52. In short, the stent 20 is less likely to buckle out of plane during compression due to its unique design.

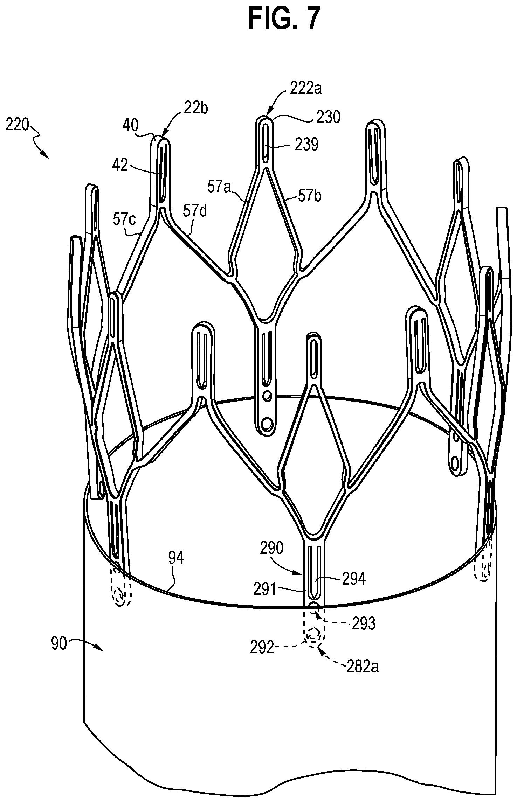

As noted above, the stent 20 may be used alone, or may be coupled to the graft 90 as shown in FIG. 2. The stent 20 may be coupled to the graft 90 using sutures, which may be disposed around portions of the stent 20 such as segments 57a-57d and/or strut 62, or the stent 20 may incorporate suture bores similar to bores 292 of FIG. 7. In any case, if the stent 20 is coupled to the graft 90 to form a stent-graft, and if the stent 20 is the most proximal stent overlapping the graft material 90 as shown in FIG. 2, then further advantages are achieved.

As one exemplary advantage, the stent 20 provides enhanced radial support to hold the proximal edge 94 of the graft 90 in an open configuration with a reduced risk of infolding, particularly due to an increased number of struts at or adjacent to the proximal edge 94. In the example of FIG. 2, six diamond-shaped cells 70 are provided (under the proximal apices 22a) and six open cells are provided (under the proximal apices 22b), and therefore twenty-four total angled strut segments 57 are provided at or adjacent the proximal edge 94, as depicted in FIG. 2. Due in part to the varying wider and narrower angles of the struts in the expanded state, an increased number of struts may be provided within the same vessel diameter, thus improving the number of contact points at the proximal edge 94 and reducing the likelihood of infolding of the graft.

As a further advantage, in the embodiment of FIG. 2, separate sealing and attachment stents are not required to hold the graft open and secure it to a vessel wall, respectively. In conventional devices, a sealing stent may fully overlap with the graft 90 along its proximal region 92 to hold it open, and an attachment stent may have a distal end secured to the proximal edge 94 of the graft 90 but the majority of its length is uncovered beyond the proximal edge 94 to anchor to a vessel wall. These separate stents may span about 40-50 mm total in length. In the embodiment of FIGS. 1-3, the stent 20 performs both of these functions, with an overall axial length that will be significantly less than providing two separate stents. For example, in some embodiments, the stent 20 may span about 15-25 mm total in length.

Therefore, the stent 20 may be suitable for a wider population, including those with smaller "landing zones" of healthy tissue, e.g., just beyond an aneurysm and prior to a branch vessel. It is expected that the required "landing zone" will be reduced by at least 50% when using the stent 20 at the proximal edge 94 of the graft 90, when compared to using separate sealing and attachment stents. Moreover, a stent-graft incorporating the stent 20 is expected to tolerate a more tortuous anatomy due to the reduced length using a single stent near the proximal edge 94, as compared to using separate sealing and attachment stents. As a related advantage, the reduced axial lengths of the connection struts 62, as part of the axially asymmetric diamond-shaped cells 70, help reduce the overall length of the stent 20.

Notably, an all diamond configuration of the stent 20 is not preferred in a tortuous anatomy or in a highly angulated vessel due to the lack of flexibility (and increased profile) of diamonds at every cell. The design of stent 20 is considerably more flexible than the all diamond configuration as the zig-zag portions 52 provide more flexible regions.

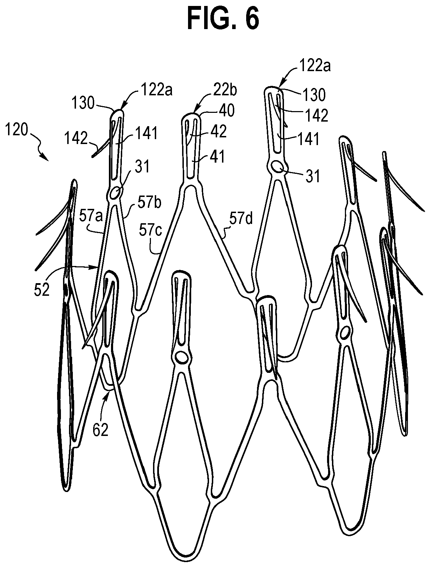

As depicted further in FIGS. 6-7, the general structure of the stent 20 may be maintained, while components near the proximal or distal apices may be changed, omitted or added. Such variations, which may tailor the stent 20 or associated stent-graft to particular applications, are within the scope of the present embodiments.

Referring now to FIG. 6, another stent 120 is one example of a modified stent that applies the general structure of the stent 20 above. The primary difference of the stent 120 is that alternating proximal apices 122a differ from proximal apices 22a of stent 20 by having barbs 142 disposed adjacent to the bore 31 housing the imaging element. The barbs 142 may be integrally formed in end regions 130 by forming slits 141, similar to the integral barbs 42 explained above. In FIG. 6, the bore 31 housing the imaging element may help identify the precise location of the proximal edge 94 of the graft 90, while the barbs 142 extend beyond the proximal edge 94 of the graft 90 to engage tissue.

Referring now to FIG. 7, another stent 220 is similar to the stents 20 and 120 described above, but may be particularly suitable for use as an attachment stent disposed at the proximal edge 94 of the graft 90 (it is noted that only a frontal portion of the graft 90 is shown for illustrative purposes, not its full circumference). The stent 220 has the basic central structure of the stent 20, but selected proximal and distal apices are modified for the particular application.

In this example, alternating proximal apices 222a comprise end regions 230 having trigger wire bore 239. In this embodiment, the stent 220 may be delivered to a target site in a compressed state using a plurality of trigger wires, where a single trigger wire may be looped through a bore 239 of each first proximal apex 222a to restrain the stent 20 during delivery. In this embodiment, trigger wires are not coupled to the second proximal apices 22b, which comprise the barbs 42. By restraining selected ones of the proximal apices, such as each first proximal apex 222a, the adjacent second proximal apices 22b also may be indirectly pulled in a radially inward direction during delivery. The configuration of the stent 220 facilitates the indirect compression of the adjacent second proximal apices 22b. Advantageously, since only selected ones of the proximal apices are restrained during delivery, the number of trigger wires may be reduced.

Referring still to FIG. 7, the stent 220 comprises distal apices 282a formed by leg regions 290, which comprise a body portion 291 having a suture bore 292, an imaging bore 293, and a barb 294 formed therein. The leg regions 290 may be coupled to the proximal edge 94 of graft 90, as depicted in FIG. 7, using one or more sutures that are looped through the graft 90 and the bores 292 of the stent 220. In this manner, the stent 220 may be used as an attachment stent for endovascular graft fixation. For example, the graft 90 may overlap with an aneurysm to seal off fluid flow into the aneurysm, while the proximal end of the stent 220 may extend in a proximal direction away from the graft 90, *e.g.,* to engage a healthy portion of a vessel wall away from a diseased portion of the aneurysm.

The imaging bore 293 of the leg region 290 may be disposed between the suture bore 292 and the barb 294, as seen in FIG. 7. The imaging bore 293 may receive any suitable radiopaque marker, such as a gold marker. In use, the imaging bores 293 may be aligned with the proximal edge 94 of the graft 90, as depicted in FIG. 7, in a manner that is particularly well-suited when the stent 220 is used for endovascular graft fixation. More specifically, the suture bore 292 overlaps with a proximal region of the graft 90, thereby allowing a suture to couple the stent 220 to the graft 90 with some desired degree of overlap. The proximal edge 94 of the graft 90 therefore may be aligned precisely with the imaging bores 293, as shown in FIG. 7. Advantageously, a physician may know exactly where the proximal edge 94 of the graft 90 is being placed because he or she can view the position of the radiopaque markers in the imaging bores 293. Therefore, the chances of inadvertently overlapping the graft material with a branch vessel, or another undesired location, may be reduced.

As will be appreciated, further embodiments may comprise various combinations of suture holes for graft couplings, marker bores for imaging elements, trigger wire holes for receiving trigger wires, and barbs. Moreover, such components may be associated with either proximal and/or distal apices of the stents 20, 120 and 220 in a manner tailored for needs of a given procedure. In some embodiments, e.g., where the stent entirely overlaps with graft material, barbs may be omitted entirely.

Although the present embodiments have generally been described as having the zig-zag portion 52 being closer to the "proximal region" of the stent 20, and the connection strut 62 being closer to the "distal region," this is for references purposes only to facilitate relative discussion of parts. The stent 20 may be placed in a bodily passageway such that the zig-zag portion 52 is at the inflow end while the connection strut 62 is at the outflow end (as generally explained in the exemplary embodiments herein), or may be disposed vice-versa such that the connection strut 62 is at the inflow end while the zig-zag portion is at an outflow end.

While various embodiments of the invention have been described, the invention is not to be restricted except in light of the attached claims and their equivalents. Moreover, the advantages described herein are not necessarily the only advantages of embodiments of the invention and it is not necessarily expected that every embodiment of the invention will achieve all of the advantages described.

All optional and preferred features and modifications of the described embodiments and dependent claims are usable in all aspects of the invention taught herein. Furthermore, the individual features of the dependent claims, as well as all optional and preferred features and modifications of the described embodiments are combinable and interchangeable with one another.

The disclosures in United States patent application number 62/808,608, from which this application claims priority, and in the abstract accompanying this application are incorporated herein by reference.

## Claims

1. A stent for use in a medical procedure, the stent comprising:
a proximal segment comprising a zig-zag portion, wherein the zig-zag portion comprises a plurality of proximal and distal apices separated by a plurality of angled strut segments, where the zig-zag portion extends circumferentially around a full perimeter of the stent;
a distal segment comprising a connection strut, where the connection strut comprises a bent segment extending between adjacent distal apices of the zig-zag portion;
at least one diamond-shaped closed cell, which is bounded proximally by first and second angled strut segments of the zig-zag portion meeting at a first proximal apex of the zig-zag portion, and further bounded distally by the connection strut having the bent segment; and
at least one distal open region, located distally beneath a second proximal apex of the zig-zag portion that is adjacent to the first proximal apex of the zig-zag portion.

2. The stent of claim 1 where, in an expanded state, the at least one diamond-shaped closed cell comprises an asymmetric shape, such that the first and second angled strut segments are different than a mirror image of the connection strut.

3. The stent of claim 1 or 2 where, in an expanded state, a first axial length spanned between the proximal and distal apices of the zig-zag portion is greater than a second axial length spanned by the connection strut when measured along a longitudinal axis of the stent.

4. The stent of any preceding claim , where at least one of the plurality of angled strut segments of the zig-zag portion comprises a first thickness, which is greater than a second thickness of the connection strut.

5. The stent of any preceding claim, where third and fourth angled strut segments of the zig-zag portion, which extend directly from the second proximal apex, comprise thicknesses greater than the connection strut.

6. The stent of any preceding claim, where the first and second angled strut segments associated with the diamond-shaped cells are orientated at a first angle relative to a longitudinal axis of the stent in an expanded state, and where third and fourth angled strut segments extending from the second proximal apex are orientated at a second angle relative to the longitudinal axis of the stent in the expanded state, where the first angle is less than the second angle.

7. The stent of claim 6, where the first angle is between about 5 degrees and about 45 degrees, and the second angle is between about 20 degrees and about 90 degrees.

8. The stent of any preceding claim, wherein the connection strut comprises one of a V-shape or a U-shape.

9. The stent of any preceding claim, where the stent comprises alternating proximal apices having different features, where a first proximal apex comprises a bore sized to receive an imaging element, and a second proximal apex comprises at least one barb.

10. The stent of any preceding claim, where the stent comprises alternating proximal apices having different features, where a first proximal apex comprises a bore sized to receive a trigger wire, and a second proximal apex comprises at least one barb.

11. The stent of any preceding claim, where the stent comprises at least one distal apex adapted to be coupled to a graft, the at least one distal apex comprising a suture bore, an imaging bore, and a barb.

12. A stent for use in a medical procedure, the stent comprising:
a proximal segment comprising a zig-zag portion, wherein the zig-zag portion comprises a plurality of proximal and distal apices separated by a plurality of angled strut segments, where the zig-zag portion extends circumferentially around a full perimeter of the stent;
a distal segment comprising a connection strut, where the connection strut comprises a bent segment extending between adjacent distal apices of the zig-zag portion; and
at least one diamond-shaped closed cell, which is bounded proximally by first and second angled strut segments of the zig-zag portion meeting at a first proximal apex of the zig-zag portion, and further bounded distally by the connection strut having the bent segment,
where, in an expanded state, the at least one diamond-shaped closed cell comprises an asymmetric shape, such that the first and second angled strut segments are different than a mirror image of the connection strut.

13. The stent of claim 12, further comprising at least one distal open region, located distally beneath a second proximal apex of the zig-zag portion that is adjacent to the first proximal apex of the zig-zag portion.

14. The stent of claim 12 or 13, where a first axial length spanned between the proximal and distal apices of the zig-zag portion is greater than a second axial length spanned by the connection strut when measured along a longitudinal axis of the stent.

15. The stent of claim 12, 13 or 14, where at least of the plurality of angled strut segments of the zig-zag portion comprises a first thickness, which is greater than a second thickness of the connection strut.
